# EUROPEAN PATENT SPECIFICATION

(11) **EP 0 817 851 B1**
(45) Date of publication and mention of the grant of the patent: **18.02.2004**
(21) Application number: 96909743.5
(22) Date of filing: 18.03.1996
(51) Int. Cl.: C12N 15/37, C07K 14/025, C07K 16/08, A61K 39/12

(54) **DNA ENCODING HUMAN PAPILLOMA VIRUS TYPE 18**
FÜR MENSCHLICHES PAPILLOMAVIRUS STAMM 18 KODIERENDE DNS
ADN CODANT LE VIRUS DU PAPILLOME DE TYPE 18 HUMAIN

(30) Priority: 22.03.1995 US 408669; 22.03.1995 US 409122
(43) Date of publication of application: 14.01.1998
(73) Proprietor: MERCK & CO., INC., Rahway, New Jersey 07065 (US)
(72) Inventor: HOFMANN, Kathryn, J., Rahway, NJ 07065 (US); JANSEN, Kathrin, U., Rahway, NJ 07065 (US); NEEPER, Michael, P., Rahway, NJ 07065 (US); JOYCE, Joseph, G., Rahway, NJ 07065 (US); GEORGE, Hugh, A., Rahway, NJ 07065 (US)
(74) Representative: Hiscock, Ian James
(86) International application number: PCT/US1996/003649
(87) International publication number: WO 1996/029413

(56) References cited:
- EP-A- 0 256 321
- EP-A- 0 456 197
- WO-A-93/02184
- WO-A-94/00152
- WO-A-94/05792
- WO-A-94/20137
- J MOL BIOL, FEB 20 1987, 193 (4) P599-608, ENGLAND, XP000603018 COLE ST ET AL: "Nucleotide sequence and comparative analysis of the human papillomavirus type 18 genome. Phylogeny of papillomaviruses and repeated structure of the E6 and E7 gene products."

## Description

### FIELD OF THE INVENTION

The present invention is directed to DNA molecules encoding purified human papillomavirus type 18 and derivatives thereof.

### BRIEF DESCRIPTION OF THE DRAWINGS

Figure 1 shows the HPV18 L1 nucleotide and deduced amino acid sequences.
Figure 2 is a list of amino acid variations within the L 1 protein of HPV 18.
Figure 3 shows the HPV18 L2 nucleotide and deduced amino acid sequences.
Figure 4 shows an immunoblot of HPV 18 L I protein expressed in yeast.
Figure 5 shows an immunoblot of HPV18 L2 protein expressed in yeast.
Figure 6 is an electron micrograph of virus-like particles formed by HPV18 L1 protein expressed in yeast.

### BACKGROUND OF THE INVENTION

Papillomavirus (PV) infections occur in a variety of animals. including humans, sheep, dogs, cats, rabbits, monkeys, snakes and cows. Papillomaviruses infect epithelial cells, generally inducing benign epithelial or fibroepithelial tumors at the site of infection. PV are species specific infective agents; a human papillomavirus does not infect a nonhuman animal.

Papillomaviruses may be classified into distinct groups based on the host that they infect. Human papillomaviruses (HPV) are further classified into more than 70 types based on DNA sequence homology. PV types appear to be type-specific immunogens in that a neutralizing immunity to infection by one type of papillomavirus does not confer immunity against another type of papillomavirus.

In humans, different HPV types cause distinct diseases. HPV types 1, 2, 3, 4, 7, 10 and 26-29 cause benign warts in both normal and immunocompromised individuals. HPV types 5, 8, 9, 12, 14, 15, 17, 19-25, 36 and 46-50 cause flat lesions in immunocompromised individuals. HPV types 6, 11, 34, 39, 41-44 and 51-55 cause benign condylomata of the genital or respiratory mucosa. HPV types 16 and 18 cause epithelial dysplasia of the genital mucosa and are associated with the majority of *in situ* and invasive carcinomas of the cervix, vagina, vulva and anal canal.

Papillomaviruses are small (50-60 nm), nonenveloped, icosahedral DNA viruses that encode for up to eight early and two late genes. The open reading frames (ORFs) of the virus genomes are designated E1 to E7 and L1 and L2, where "E" denotes early and "L" denotes late. L1 and L2 code for virus capsid proteins. The early (E) genes are associated with functions such as viral replication and cellular transformation.

The L 1 protein is the major capsid protein and has a molecular weight of 55-60 kDa. The L2 protein is a minor capsid protein which has a predicted molecular weight of 55-60 kDa and an apparent molecular weight of 75-100 kDa as determined by polyacrylamide gel electrophoresis. Immunological data suggest that most of the L2 protein is internal to the L1 protein within the viral capsomere. The L1 ORF is highly conserved among different papillomaviruses. The L2 proteins are less conserved among different papillomaviruses.

The L1 and L2 genes have been identified as good targets for immunoprophylaxis. Studies in the cottontail rabbit papillomavirus (CRPV) and bovine papillomavirus (BPV) systems have shown that immunizations with the L1 and L2 proteins expressed in bacteria or by using vaccinia vectors protected animals from viral infection. Expression of papillomavirus L1 genes in baculovirus expression systems or using vaccinia vectors resulted in the assembly of virus-like particles (VLP) which have been used to induce high-titered virus-neutralizing antibody responses that correlate with protection from viral challenge.

Following HPV type 16, HPV 18 is the second most prevalent HPV type found in cervical carcinomas. HPV 18 was detected in 5-20% of cervical cancer biopsies collected from various parts of the world (Ikenberg, H. 1990. Human papillomavirus DNA in invasive genital carcinomas. In Genital Papillomavirus Infections, G. Gross *et al.,* eds. p. 85-112). There appears to be a geographic dependence of infection with HPV 18 since tumor biopsies from African and South American women harbor HPV 18 more frequently than similar biopsies from European and North American women. The underlying reasons for these geographic differences are not known. The development of a vaccine against HPV 18 infection becomes extremely relevant since HPV 18 is also associated with more aggressively growing cancers and is rarely found in the milder precursor lesions, CIN I-II.

### SUMMARY OF THE INVENTION

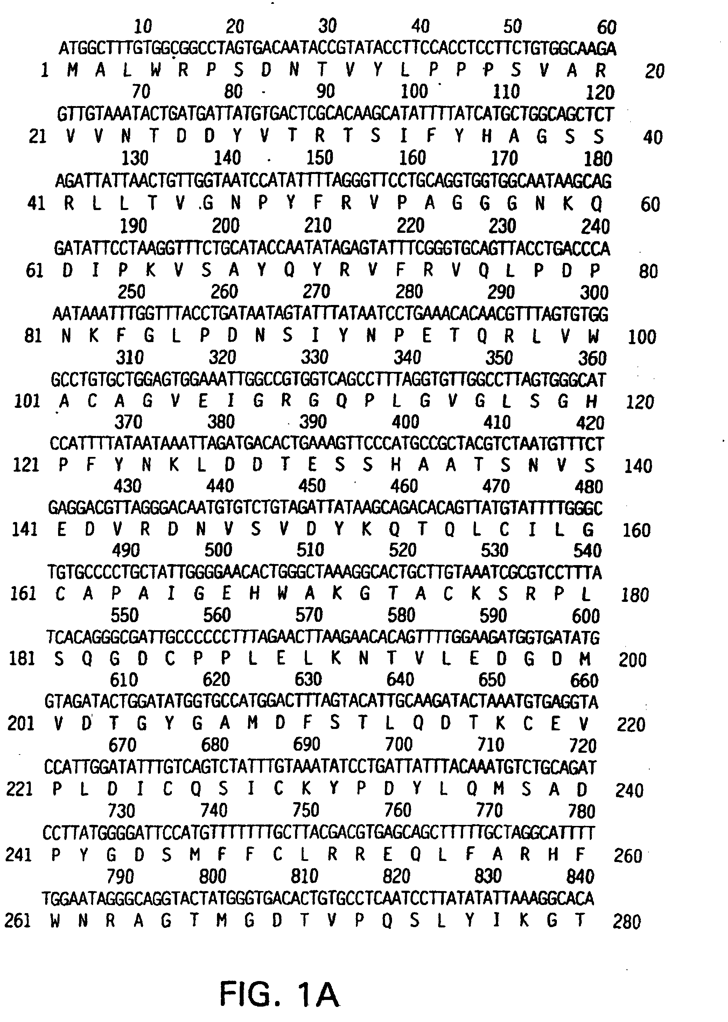

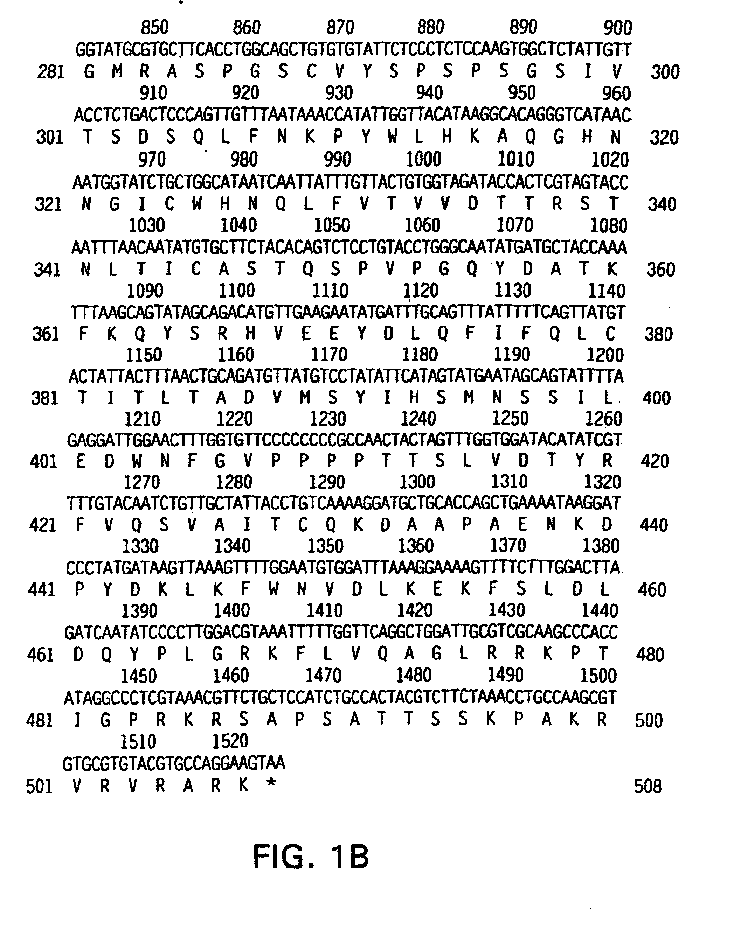

The present invention is directed to DNA molecules encoding purified human papillomavirus type 18 (HPV type 18; HPV18) L1 and L2 proteins shown in Fig. 1 and and uses of the DNA molecules.

### DETAILED DESCRIPTION OF THE INVENTION

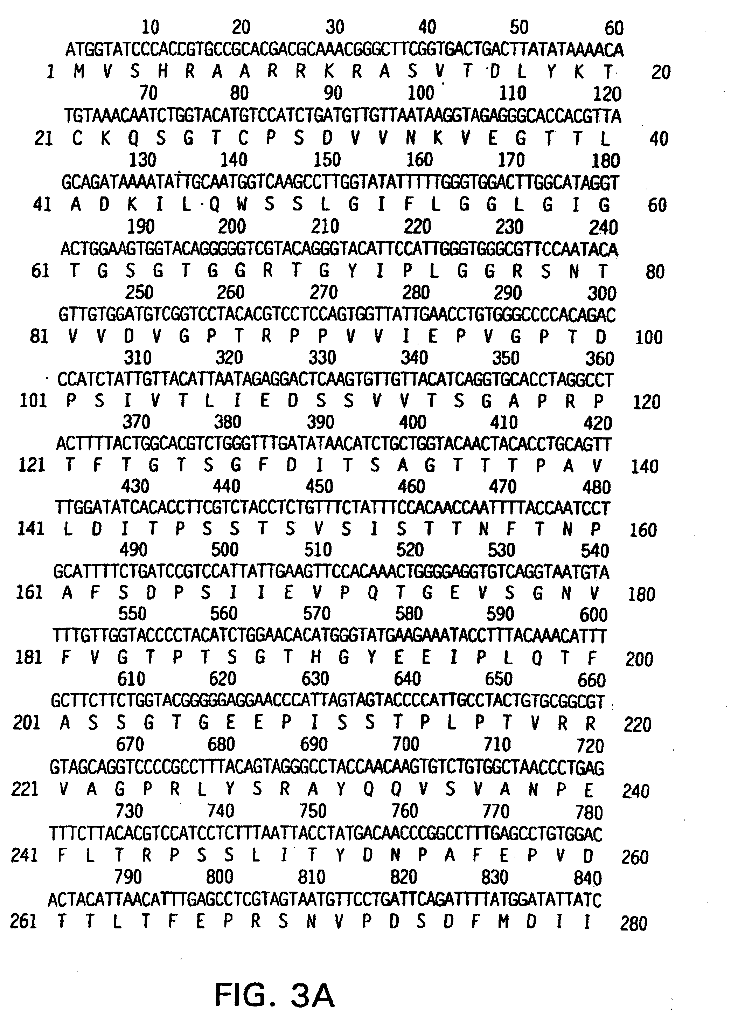

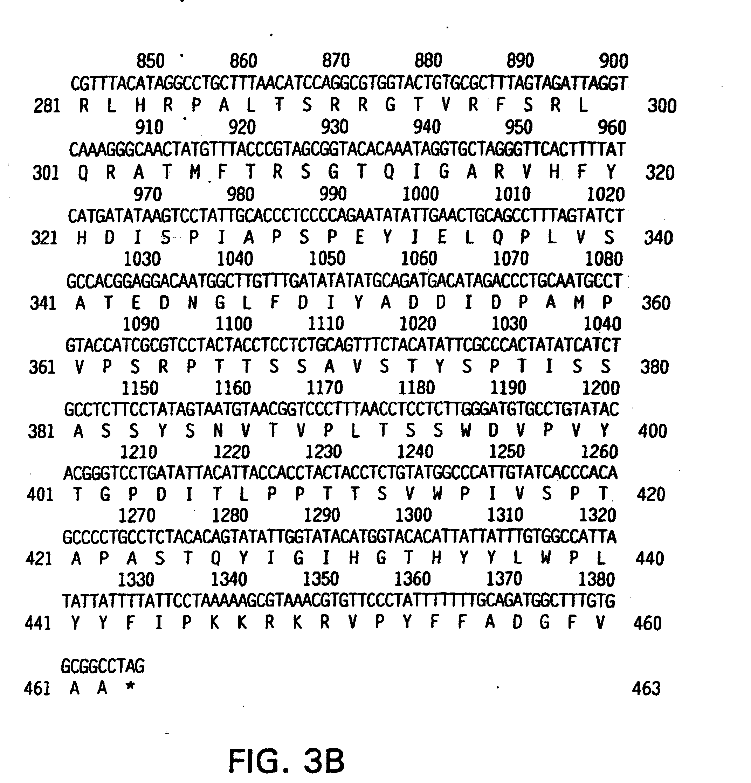

The present invention is directed to DNA molecules encoding purified human papillomavirus type 18 (HPV type 18: HPV18) L1 and Fig.3. L2 proteins shown in Fig. 1 and Fig. 3. peptides and proteins encoded by the DNA, vaccines comprising the DNA or vaccines comprising proteins encoded by the DNA. immunological compositions comprising the DNA or the proteins encoded by the DNA. kits containing the DNA or RNA derived from the DNA or proteins encoded by the DNA.

Pharmaceutically useful compositions comprising the DNA or proteins shown in Fig. 1 and Fig. 3 may be formulated according to known methods such as by the admixture of a pharmaceutically acceptable carrier. Examples of such carriers and methods of formulation may be found in Remington's Pharmaceutical Sciences. To form a pharmaceutically acceptable composition suitable for effective administration, such compositions will contain an effective amount of the DNA or protein or VLP. Such compositions may contain DNA or proteins or VLP derived from more than one type of HPV.

Therapeutic or diagnostic compositions of the invention are administered to an individual in amounts sufficient to treat or diagnose PV infections. The effective amount may vary according to a variety of factors such as the individual's condition, weight, sex and age. Other factors include the mode of administration. Generally, the compositions will be administered in dosages ranging from about 1 µg to about 1 mg.

The pharmaceutical compositions may be provided to the individual by a variety of routes such as subcutaneous, topical, oral, mucosal, intravenous and intramuscular.

The vaccines of the invention comprise DNA, RNA or proteins encoded by the DNA that contain the antigenic determinants necessary to induce the formation of neutralizing antibodies in the host. Such vaccines are also safe enough to be administered without danger of clinical infection; do not have toxic side effects; can be administered by an effective route; are stable; and are compatible with vaccine carriers.

The vaccines may be administered by a variety of routes. such as orally, parenterally, subcutaneously, mucosally, intravenously or intramuscularly. The dosage administered may vary with the condition. sex. weight, and age of the individual: the route of administration: and the type PV of the vaccine. The vaccine may be used in dosage forms such as capsules, suspensions, elixirs, or liquid solutions. The vaccine may be formulated with an immunologically acceptable carrier.

The vaccines are administered in therapeutically effective amounts, that is, in amounts sufficient to generate a immunologically protective response. The therapeutically effective amount may vary according to the type of PV. The vaccine may be administered in single or multiple doses.

The DNA of the present invention may be used in the formulation of immunogenic compositions. Such compositions, when introduced into a suitable host, are capable of inducing an immune response in the host.

The DNA of the present invention may be used to serotype HPV infection and HPV screening. The DNA, recombinant proteins, VLP lend themselves to the formulation of kits suitable for the detection and serotyping of HPV. Such a kit would comprise a compartmentalized carrier suitable to hold in close confinement at least one container. The carrier would further comprise reagents such as HPV18 DNA, recombinant HPV protein or VLP or anti-HPV antibodies suitable for detecting a variety of HPV types. The carrier may also contain means for detection such as labeled antigen or enzyme substrates or the like.

The DNA and derived proteins shown in Figs 1 and 3 therefrom are also useful as molecular weight and molecular size markers.

Because the genetic code is degenerate. more than one codon may be used to encode a particular amino acid, and therefore. the amino acid sequence can be encoded by any of a set of similar DNA oligonucleotides. Only one member of the set will be identical to the HPV 18 sequence but will be capable of hybridizing to HPV18 DNA even in the presence of DNA oligonucleotides with mismatches under appropriate conditions. Under alternate conditions, the mismatched DNA oligonucleotides may still hybridize to the HPV 18 DNA to permit identification and isolation of HPV18 encoding DNA.

The purified HPV18 DNA of the invention may be used to isolate and purify homologues and fragments of HPV 18 from other sources. To accomplish this, the first HPV 18 DNA may be mixed with a sample containing DNA encoding homologues of HPV18 under appropriate hybridization conditions. The hybridized DNA complex may be isolated and the DNA encoding the homologous DNA may be purified therefrom.

A variety of procedures may be used to molecularly clone HPV 18 DNA. These methods include, but are not limited to, direct functional expression of the HPV 18 genes following the construction of a HPV18-containing cDNA or genomic DNA library in an appropriate expression vector system. Another method is to screen HPV18-containing cDNA or genomic DNA library constructed in a bacteriophage or plasmid shuttle vector with a labeled oligonucleotide probe designed from the amino acid sequence of the HPV 18. An additional method consists of screening a HPV18-containing cDNA or genomic DNA library constructed in a bacteriophage or plasmid shuttle vector with a partial DNA encoding the HPV 18. This partial DNA is obtained by the specific polymerase chain reaction (PCR) amplification of HPV 18 DNA fragments through the design of degenerate oligonucleotide primers from the amino acid sequence of purified HPV 18. Another method is to isolate RNA from HPV 18-producing cells and translate the RNA into protein via an *in vitro* or an *in vivo* translation system. The translation of the RNA into a peptide or a protein will result in the production of at least a portion of HPV 18 protein which can be identified by, for example, the activity of HPV 18 protein or by immunological reactivity with an anti-HPV 18 antibody. In this method. pools of RNA isolated from HPV I 8-producing cells can be analyzed for the presence of an RNA which encodes at least a portion of the HPV 18. Further fractionation of the RNA pool can be done to purify the HPV 18 RNA from non-HPV 18 RNA. The peptide or protein produced by this method may be analyzed to provide amino acid sequences which in turn are used to provide primers for production of HPV 18 cDNA, or the RNA used for translation can be analyzed to provide nucleotide sequences encoding HPV 18 and produce probes for the screening of a HPV 18 cDNA library. These methods are known in the art and can be found in, for example, Sambrook, J., Fritsch, E.F., Maniatis, T. in Molecular Cloning: A Laboratory Manual, Second Edition, Cold Spring Harbor Laboratory Press, Cold Spring Harbor, NY. 1989.

It is apparent that other types of libraries, as well as libraries constructed from other cells or cell types, may be useful for isolating HPV18-encoding DNA. Other types of libraries include, but are not limited to, cDNA libraries derived from other cells or cell lines containing HPV type 18 and genomic DNA libraries.

Preparation of cDNA libraries can be performed by a variety of techniques. cDNA library construction techniques can be found in Sambrook, J., *et al., supra.* It is apparent that DNA encoding HPV18 may also be isolated from a suitable genomic DNA library. Construction of genomic DNA libraries can be performed by a variety of techniques. Genomic DNA library construction techniques can be found in Sambrook, J., *et al. supra.*

The cloned HPV18 DNA obtained through the methods described herein may be recombinantly expressed by molecular cloning into an expression vector containing a suitable promoter and other appropriate transcription regulatory elements, and transferred into prokaryotic or eukaryotic host cells to produce recombinant HPV 18. Techniques for such manipulations are fully described in Sambrook, J., *et al., supra,* and are known in the art.

Expression vectors are defined herein as DNA sequences that are required for the transcription of cloned copies of genes and the translation of their mRNAs in an appropriate host. Such vectors can be used to express eukaryotic genes in a variety of hosts such as bacteria. bluegreen algae, plant cells, insect cells, fungal cells and animal cells. Specifically designed vectors allow the shutting of DNA between hosts such as bacteria-yeast or bacteria-animal cells or bacteria-fungal cells or bacteria-invertebrate cells. An appropriately constructed expression vector should contain: an origin of replication for autonomous replication in host cells. selectable markers, a limited number of useful restriction enzyme sites, a potential for high copy number, and active promoters. A promoter is defined as a DNA sequence that directs RNA polymerase to bind to DNA and initiate RNA synthesis. A strong promoter is one which causes mRNAs to be initiated at high frequency. Expression vectors may include, but are not limited to, cloning vectors, modified cloning vectors, specifically designed plasmids or viruses.

A variety of mammalian expression vectors may be used to express the HPV18 DNA in mammalian cells. Commercially available mammalian expression vectors which may be suitable for recombinant HPV 18 expression, include but are not limited to, pcDNA3 (Invitrogen), pMC1neo (Stratagene), pXT1 (Stratagene), pSG5 (Stratagene), EBO-pSV2-neo (ATCC 37593) pBPV-1 (8-2) (ATCC 37110), pdBPV-MMTneo(342-12) (ATCC 37224), pRSVgpt (ATCC 37199), pRSVneo (ATCC 37198), pSV2-dhfr (ATCC 37146), pUCTag (ATCC 37460), and λZD35 (ATCC 37565).

A variety of bacterial expression vectors may be used to express the HPV18 DNA in bacterial cells. Commercially available bacterial expression vectors which may be suitable include, but are not limited to pET11a (Novagen), lambda gt11 (Invitrogen), pcDNAII (Invitrogen), pKK223-3 (Pharmacia).

A variety of fungal cell expression vectors may be used to express the HPV18 in fungal cells. Commercially available fungal cell expression vectors which may be suitable include but are not limited to pYES2 (Invitrogen), *Pichia* expression vector (Invitrogen), and *Hansenula* expression (Rhein Biotech, Dusseldorf, Germany).

A variety of insect cell expression vectors may be used to express the HPV18 DNA in insect cells. Commercially available insect cell expression vectors which may be suitable include but are not limited to pBlue Bac III (Invitrogen) and pAcUW51 (PharMingen, Inc.).

An expression vector containing the DNA encoding HPV 18 shown in Fig 1 and Fig 3 may be used for expression of the HPV18 proteins in a cell, tissues, organs, or animals. Host cells may be prokaryotic or eukaryotic, including but not limited to bacteria such as *E. coli,* fungal cells such as yeast, mammalian cells including but not limited to cell lines of human, bovine, porcine, monkey and rodent origin, and insect cells including but not limited to Drosophila and silkworm derived cell lines. Cell lines derived from mammalian species which may be suitable and which are commercially available, include but are not limited to, L cells L-M(TK-) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-K (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26) and MRC-5 (ATCC CCL 171).

The expression vector may be introduced into host cells via any one of a number of techniques including but not limited to transformation, transfection, lipofection, protoplast fusion, and electroporation. The expression vector-containing cells are clonally propagated and individually analyzed to determine whether they produce HPV18 protein. Identification of HPV 18 expressing host cell clones may be done by several means, including but not limited to immunological reactivity with anti-HPV 18 antibodies, and the presence of host cell-associated HPV 18 activity, such as HPV 18-specific ligand binding or signal transduction defined as a response mediated by the interaction of HPV18-specific ligands with the expressed HPV 18 proteins.

Expression of HPV DNA may also be performed using *in vitro* produced synthetic mRNA or native mRNA. Synthetic mRNA or mRNA isolated from HPV 18 producing cells can be efficiently translated in various cell-free systems, including but not limited to wheat germ extracts and reticulocyte extracts, as well as efficiently translated in cell based systems, including but not limited to microinjection into frog oocytes, with microinjection into frog oocytes being preferred.

Following expression of the HPV18 protein(s) in a host cell, HPV18 protein may be recovered to provide HPV18 in purified form. Several HPV 18 purification procedures are available and suitable for use. As described herein, recombinant HPV 18 protein may be purified from cell lysates and extracts by various combinations of, or individual application of salt fractionation, ion exchange chromatography, size exclusion chromatography, hydroxylapatite adsorption chromatography and hydrophobic interaction chromatography.

In addition, recombinant HPV18 may be separated from other cellular proteins by use of an immunoaffinity column made with monoclonal or polyclonal antibodies specific for full length nascent HPV 18, or polypeptide fragments of HPV18.

Kits containing the HPV18 DNA, HPV18 RNA or HPV18 protein may be prepared. Such kits are used to detect DNA which hybridizes to HPV 18 DNA or to detect the presence of HPV18 protein(s) or peptide fragments in a sample. Such characterization is useful for a variety of purposes including but not limited to forensic analyses and epidemiological studies.

For combination treatment with more than one active agent, where the active agents are in separate dosage formulations, the active agents can be administered concurrently, or they each can be administered at separately staggered times.

The dosage regimen is selected in accordance with a variety of factors including type, species, age, weight, sex and medical condition of the patient; the severity of the condition to be treated; the route of administration; the renal and hepatic function of the patient; and the particular compound thereof employed. A physician of ordinary skill can readily determine and prescribe the effective amount of the drug required to prevent, counter or arrest the progress of the condition. Optimal precision in achieving concentrations of drug within the range that yields efficacy without toxicity requires a regimen based on the kinetics of the drug's availability to target sites. This involves a consideration of the distribution, equilibrium, and elimination of a drug.

### EXAMPLE 1

### Cloning of HPV 18 genome

Total genomic DNA was prepared from the human cervical carcinoma-derived cell line, SW756 (Freedman, R. S., *et al.,* 1982, *In Vitro,* Vol 18, pages 719-726) by standard techniques. The DNA was digested with EcoRI and electrophoresed through a 0.8% low-melting temperature, agarose preparative gel. A gel slice was excised corresponding to DNA fragments approximately 12 kbp in length. The agarose was digested using Agarase™ enzyme (Boehringer Mannheim. Inc.) and the size-fractionated DNA was precipitated, dephosphorylated and ligated with EcoRI digested lambda EMBL4 arms (Stratagene, Inc.). The lambda library was packaged using Gigapack II Gold packaging extract (Stratagene, Inc.). HPV 18-positive clones were identified using a 700 bp, HPV 18 L1 DNA probe that was generated by polymerase chain reaction (PCR) using SW756 DNA as template and oligonucleotide primers that were designed based on the published HPV18 L1 DNA sequence (Cole and Danos, 1987, *J. Mol. Biol.,* Vol. 193:599-608: Genbank Accession #X05015). A HPV18-positive. lambda clone was isolated that contained a 12 kbp EcoRI fragment insert and was designated as #187-1.

### EXAMPLE 2

### Construction of Yeast Expression Vectors

The HPV18 L1 open reading frame (ORF) was amplified by PCR using clone #187-1 as template, Vent polymerase™ (New England Biolabs, Inc.), 10 cycles of amplification (94°C, 1 min; 50°C, 1 min; 72°C, 2 min) and the following oligonucleotide primers which contain flanking BgIII sites (underlined):
sense primer, 
5'-GAAGATCTCACAAAACAAAATGGCTTTGTGG
CGGCCTAGTG-3',
 antisense primer, 
5'-GAAGATCTTTACTTCCTGGCACGTAC
ACGCACACGC-3'.
 The sense primer introduces a yeast non-translated leader sequence (Kniskern, *et al.,* 1986*, Gene,* Vol. 46:135-141) immediately upstream to the HPV 18 L 1 initiating methionine codon (highlighted in bold print). The 1.5 kbp L1 PCR product was digested with BglII and gel purified.

The pGAL1-10 yeast expression vector was constructed by isolating a 1.4 kbp SphI fragment from a pUC18/bidirectional GAL promoter plasmid which contains the *Saccharomyces cerevisiae* divergent *GAL1-GAL10* promoters from the plasmid pBM272 (provided by Mark Johnston, Washington University, St. Louis, MO). The divergent promoters are flanked on each side by a copy of the yeast *ADH1* transcriptional terminator, a BamHI cloning site located between the *GAL1* promoter and the first copy of the *ADH1* transcriptional terminator and a SmaI cloning site located between the *GAL10* promoter and the second copy of the *ADH1* transcriptional terminator. A yeast shuttle vector consisting of pBR322, the yeast *LEU2d* gene, and the yeast *2u* plasmid (gift of Benjamin Hall, University of Washington. Seattle. WA) was digested with SphI and ligated with the 1.4 kbp SphI divergent *GAL* promoter fragment resulting in pGAL1-10. pGAL1-10 was linearized with BamHI which cuts between the *GAL1* promoter and the *ADH1* transcription terminator. The BamHI digested vector and the BgIII digested HPV 18 L1 PCR fragment were ligated and used to transform *E. coli* DH5 cells (Gibco BRL, Inc.). A pGAL1-10 plasmid was isolated which contains the HPV18 L1 gene and was designated p191-6.

A yeast expression vector that co-expresses both the HPV 18 L1 and L2 genes was constructed. Plasmid p191-6 (pGAL1-10 + HPV18 L1) was digested with SmaI which cuts between the *GAL10* promoter and the *ADH1* transcription terminator. The 1.4 kbp HPV 18 L2 gene was amplified by PCR as described above using the following oligonucleotide primers which contain flanking SmaI sites (underlined):
sense primer, 
5'-TCCCCCGGGCACAAAACAAAATG
GTATCCCACCGTGCCGCACGAC-3',
 antisense primer, 5'-TCCCCCGGGCTAGGCCGCCACAAAGCCATCTGC-3'. The sense primer introduces a yeast non-translated leader sequence (Kniskern *et al.,* 1986, *supra)* immediately upstream to the HPV18 L2 initiating methionine codon (highlighted in bold print). The PCR fragment was digested with SmaI, gel purified and ligated with the SmaI digested p191-6 plasmid. A pGAL1-10 plasmid containing both the HPV18 L1 and L2 genes was isolated and designated, p 195-11.

### EXAMPLE 3

### Typing of Clinical Samples

Cervical biopsy samples were collected at the Veterans Administration Medical Center in Indianapolis, IN (courtesy of Dr. Darron Brown) and at the Albert Einstein Medical Center in Philadelphia. PA (courtesy of Dr. Joan Adler) and were frozen at - 20°C. DNA was isolated as described by Brown *et al.,* 1993 (Brown, D. *et al.,* 1993. *J. Clin. Microbiol.,* Vol. 31:2667-2673). Briefly. clinical specimens were processed with a Braun mikro-dismembrator II (B. Braun Instruments. Melsungen, Germany) and solubilized in buffer containing 10 mM EDTA and 0.6% (w/v) sodium dodecyl sulfate (SDS). Samples were adjusted to 20 mM Tris pH 7.4 and protein was digested with 50 mcg/mL Proteinase K in the presence of 0.1 mcg/mL RNase A followed by extraction with phenol/chloroform/isoamyl alcohol. DNA was ethanol precipitated and quantified by spectrophotometry.

The DNA samples were screened for the presence of HPV18 by PCR and Southern blot analyses. A 256 bp segment of the HPV18 L1 ORF was amplified by PCR using the following oligonucleotide primers:
sense primer, 5'-CAATCCTTATATATTAAAGGCACAGGTATG-3', antisense primer, 5'-CATCATATTGCCCAGGTACAGGAGACTGTG-3'. The PCR conditions were according to the manufacturer's recommendations for the AmpliTaq™ DNA Polymerase/GeneAmp™ kit (Perkin Elmer Corp.) except that 0.5 µl of clinical sample DNA was used as template and 10 pmoles of each primer, 2 mM dNTPs and 2.0 mM MgCl₂ were in the final reaction mixture. A 2 min, 94°C denaturation step was followed by 40 cycles of amplification (94°C, 1 min; 45°C, 1 min; 72°C, 1 min).

The PCR products were electrophoresed through a 3.0% agarose gel, blotted onto nylon membranes and hybridized with a ³²P-labeled HPV18 L1-specific oligonucleotide probe.

### EXAMPLE 4

### DNA Sequencing of L1 and L2 genes

The HPV18 L1 and L2 genes in clones #187-1, p191-6 and p 195-11 were sequenced using the PRIZM Sequencing kit and the automated DNA ABI Sequencer #373A (Applied Biosystems). To obtain a consensus HPV18 sequence, portions of the L1 gene DNA were amplified by PCR from human clinical isolates, sequenced and compared to the claimed and published sequences. A 256 bp fragment (nucleotides 817-1072) was amplified from each clinical DNA isolate for this purpose using the oligonucleotides and heating cycles described in Example 3. The following primers.
5'-GAAGATCTCACAAAACAAAATGGCTTTGTGGCGGCCTAGTG
3' and 5'- CCTAACGTCCTCAGAAACATTAGAC-3' were used to amplify an amino-terminal 432 bp portion of L1 DNA (nucleotides 1-431) using the heating cycles described in Example 3. Both PCR products were ligated separately with plasmid pCRII (Invitrogen Corp.) using the reagents and procedures recommended by the manufacturer. Plasmid DNA was isolated from the transformants and those containing EcoRI inserts were sequenced.

### EXAMPLE 5

### Analysis of DNA and Deduced Amino Acid Sequences

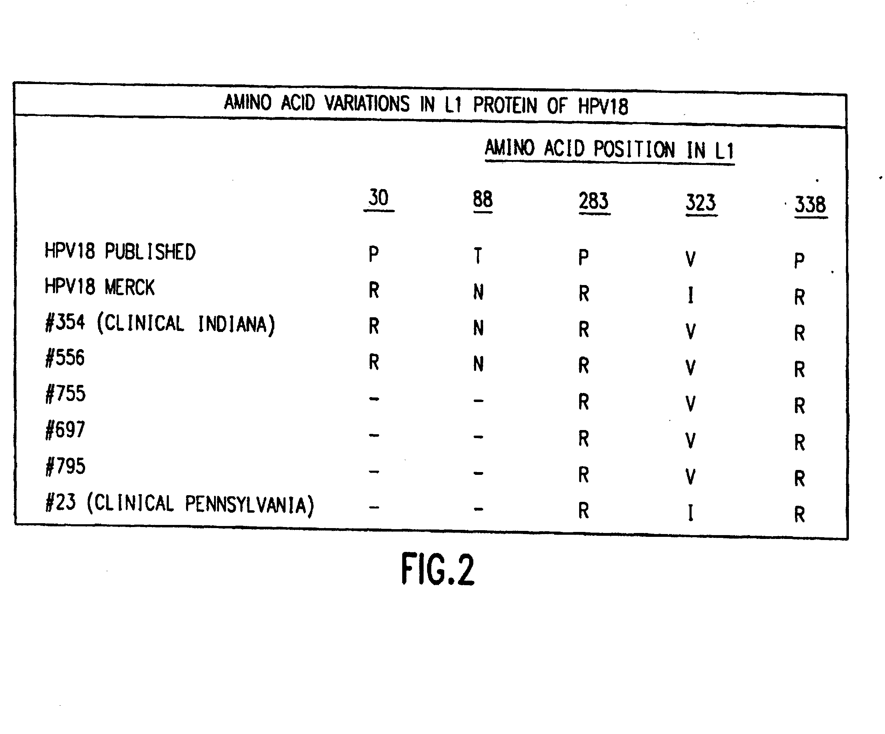

The nucleotide and deduced amino acid (aa) sequences of the claimed HPV18 L1 are shown in Figure 1. The DNA sequence was derived from a consensus of clones #187-1, p191-6 and p195-11. A comparison of the claimed HPV18 L1 nucleotide sequence with the published HPV18 L1 sequence (Genbank Accession #X05015) identified 20 bp changes out of 1524 bps. Five of the nucleotide changes (C to G at position 89, C to A at 263, C to G at 848, G to A at 967 and C to G at 1013) result in amino acid substitutions. The five residue differences from published are P to R at aa positions 30, 283 and 338, T to N at aa 88 and V to I at aa 323 (Figure 2). Positions 88 and 323 represent conservative changes while the three P to R changes may substantially alter the physical properties of the expressed L1 protein.

A comparison of the amino acid sequences derived from clinical isolates (numbers 354, 556, 755, 697, 795 and 23) with the claimed sequence and the published sequence is shown in Figure 2. There are four locations where the clinical isolates and the claimed sequence differ from the published sequence. Positions 30, 283 and 338 encode arginine (R) in all the isolates found to date, including the claimed sequence. This is in sharp contract to the published sequence which has prolines (P) at each of these locations. Furthermore, position 88 is an asparagine (N) in the isolates and the claimed sequence but is a threonine (T) in the published sequence. The last difference. position 323, was found to be a valine (V) in many of the clinical isolates and the published strain versus an isoleucine (I) in the claimed sequence and one of the isolates (#23). The conclusion is that the claimed sequence reflects the predominant viral sequences that are associated with clinical infections and the absence of isolates containing any of the position 30, 283 or 338 prolines of the published sequence suggests that the published clone is either an artefact or an inconsequential subtype.

The nucleotide and deduced aa sequences of HPV18 L2 were derived from a consensus sequence of clones #187-1 and p195-11 and are shown in Figure 3. A comparison of the L2 nucleotide sequence with the published HPV18 sequence (Genbank Accession #X05015) identified 40 bp changes out of 1389 bps. The bp differences result in 14 changes at the aa level: P to S at aa 29, P to N at aa 33, A to S at aa 177, D to E at aa 266, D to N at aa 270, D to G at aa 346, M to I at 355, V to M at aa 359, S to P at aa 365, Fto S at aa 369, Fto V at aa 371, F to S at aa 372, K to T at aa 373 and S to P at aa 409.

### EXAMPLE 6

### Generation of HPV18 L2 Antiserum

HPV18 L2 specific antibodies were prepared in goats using a *trpE*-HPV18 L2 fusion protein expressed in *E. coli.* The full-length L2 ORF was amplified by PCR using oligonucleotide primers providing HindIII and BamHI sites flanking the 5'- and 3'- ends, respectively. The L2 fragment was inserted into the HindIII-BamHI digested, pATH23 expression plasmid (Koerner *et al.,* 1991, *Meth. Enzymol.* Vol. 194:477-490). The fusion protein was expressed in *E. coli* RR1 cells (Gibco BRL. Inc.) after induction with 3-b-indoleacrylic acid. The insoluble fraction was analyzed by SDS-PAGE followed by staining with Coomassie Blue. The *trpE*-L2 fusion protein accounted for the major portion of the *E. coli* insoluble fraction. Goats were immunized with the trpE-L2 fusion protein according to the standard protocol of Pocono Rabbit Farm and Laboratory, Inc. for fusion protein antigens (Protein Rabbit Farm. Canadensis. PA).

### EXAMPLE 7

### Preparation of Yeast Strain U9

*Saccharomyces cerevisiae* strain 2150-2-3 *(MATalpha, leu2-04, adeI,* cir°) was obtained from Dr. Leland Hartwell (University of Washington, Seattle, WA). Cells of strain 2150-2-3 were propagated overnight at 30°C in 5 mL of YEHD medium (Carty *et al., J. Ind Micro 2* (1987) 117-121). The cells were washed 3 times in sterile, distilled water, resuspended in 2 mL of sterile distilled water, and 0.1 mL of cell suspension was plated onto each of six 5-fluoro-orotic acid (FOA) plates in order to select for *ura3* mutants (Cold Spring Harbor Laboratory Manual for Yeast Genetics). The plates were incubated at 30°C. The medium contained per 250 mL distilled water: 3.5 g, Difco Yeast Nitrogen Base without amino acids and ammonium sulfate; 0.5 g 5-Fluoro-orotic acid; 25 mg Uracil; and 10.0 g Dextrose.

The medium was sterilized by filtration through 0.2 µm membranes and then mixed with 250 mL of 4% Bacto-Agar (Difco) maintained at 50°C, 10 mL of a 1.2 mg/mL solution of adenine, and 5 mL of L-leucine solution (180 mg/ 50 mL). The resulting medium was dispensed at 20 mL per petri dish.

After 5 days of incubation, numerous colonies had appeared. Single colonies were isolated by restreaking colonies from the initial FOA plates onto fresh FOA plates which were then incubated at 30°C. A number of colonies from the second set of FOA plates were tested for the presence of the *ura3* mutation by replica-plating onto both YEHD plates and uracil-minus plates. The desired result was good growth on YEHD and no growth on uracil-minus medium. One isolate (U9) was obtained which showed these properties. It was stored as a frozen glycerol stock (strain #325) at -70°C for later use.

### EXAMPLE 8

### Preparation of a Vector for disruption of the Yeast MNN9 gene

In order to prepare a vector for disruption of the *MNN9* gene. it was necessary to first clone the *MNN9* gene from *S. cerevisiae* genomic DNA. This was accomplished by standard Polymerase Chain Reaction (PCR) technology. A 5' sense primer and 3' antisense primer for PCR of the full-length *MNN9* coding sequence were designed based on the published sequence for the yeast *MNN9* gene (Zymogenetics: EPO Patent Application No. 88117834.7, Publication No. 0-314-096-A2). The following oligodeoxynucleotide primers containing flanking HindIII sites (underlined) were used:
sense primer: 
5'-CTT AAA GCT TAT GTC ACT TTC TCT TGT ATC
G-3'
 antisense primer:

5'-TGA TAA GCT TGC TCA ATG GTT CTC TTC
CTC-3'.

The initiating methionine codon for the MNN9 gene is highlighted in bold print. The PCR was conducted using genomic DNA from *S*. *cerevisiae* strain JRY188 as template, Taq DNA polymerase (Perkin Elmer) and 25 cycles of amplification (94°C 1 min., 37°C 2 min., 72°C 3 min.). The resulting 1.2 kbp PCR fragment was digested with HindIII, gel-purified, and ligated with HindIII-digested, alkaline-phosphatase treated pUC13 (Pharmacia). The resulting plasmid was designated p1183.

In order to disrupt the MNN9 gene with the yeast *URA3* gene, the plasmid pBR322-URA3 (which contains the 1.1 Kbp HindIII fragment encoding the *S. cerevisiae URA3* gene subcloned into the HindIII site of pBR322) was digested with HindIII and the 1.1 kbp DNA fragment bearing the functional *URA3* gene was gel-purified, made blunt-ended with T4 DNA polymerase, and then ligated with PmII-digested plasmid p1183 (PmII cuts within the MNN9 coding sequence). The resulting plasmid p 1199 contains a disruption of the *MNN9* gene by the functional *URA3* gene.

### EXAMPLE 9

### Construction of U9-derivative strain 1372 containing disruption of MNN9 gene

For disruption of the *MNN9* gene in strain U9 (#325), 30 µg of plasmid p 1199 were digested with HindIII to create a linear *mnn9::URA3* disruption cassette. Cells of strain 325 were transformed with the HindIII-digested p 1199 DNA by the spheroplast method (Hinnen *et al.,* 1978, *Proc. Natl. Acad. Sci. USA* 75:1929-1933) and transformants were selected on a synthetic agar medium lacking uracil and containing 1.0 M sorbitol. The synthetic medium contained, per liter of distilled water: Agar, 20 g; Yeast nitrogen base w/o amino acids, 6.7 g; Adenine, 0.04 g; L-tyrosine, 0.05 g; Sorbitol, 182 g; Glucose, 20 g; and Leucine Minus Solution #2, 10 ml. Leucine Minus Solution #2 contains per liter of distilled water: L-arginine, 2 g; L-histidine, 1 g; L-Leucine, 6 g; L-Isoleucine, 6 g; L-lysine, 4 g; L-methionine, 1 g; L-phenylalanine, 6 g: L-threonine, 6 g: L-tryptophan, 4 g.

The plates were incubated at 30°C for five days at which time numerous colonies had appeared. Chromosomal DNA preparations were made from 10 colonies and then digested with EcoRI plus HindIII. The DNA digests were then evaluated by Southern blots (J. Sambrook *et al.,* Molecular Cloning: A Laboratory Manual, 2nd edition. Cold Spring Harbor Laboratory Press, 1989) using the 1.2 kbp HindIII fragment bearing the *MNN9* gene (isolated from plasmid p 1199) as a probe. An isolate was identified (strain #1372) which showed the expected DNA band shifts on the Southern blot as well as the extreme clumpiness typically shown by *mnn9* mutants.

### EXAMPLE 10

### Construction of a Vector for Disruption of Yeast HIS3 Gene

In order to construct a disruption cassette in which the *S*. *cerevisiae HIS3* gene is disrupted by the *URA3* gene, the plasmid YEp6 (K. Struhl *et al.,* 1979. *Proc. Natl. Acad. Sci., USA* 76:1035) was digested with BamHI and the 1.7 kbp BamHI fragment bearing the *HIS3* gene was gel-purified, made blunt-ended with T4 DNA polymerase, and ligated with pUC18 which had been previously digested with BamHI and treated with T4 DNA polymerase. The resulting plasmid (designated p 1501 or pUC18-HIS3) was digested with NheI (which cuts in the *HIS3* coding sequence), and the vector fragment was gel-purified, made blunt-ended with T4 DNA polymerase, and then treated with calf intestine alkaline phosphatase. The *URA3* gene was isolated from the plasmid pBR322-URA3 by digestion with HindIII and the 1.1 kbp fragment bearing the *URA3* gene was gel-purified, made blunt-ended with T4 DNA polymerase, and ligated with the above pUC18-HIS3 NheI fragment. The resulting plasmid (designated pUC 18-his3::URA3 or p 1505) contains a disruption cassette in which the yeast *HIS3* gene is disrupted by the functional *URA3* gene.

### EXAMPLE 11

### Construction of Vector for Disruption of Yeast PRB1 Gene by the HIS3 Gene

Plasmid FP8ΔH bearing the *S. cerevisiae PRB1* gene was provided by Dr. E. Jones of Carnegie-Mellon Univ. (C. M. Moehle *et al.*, 1987, *Genetics* 115:255-263). It was digested with HindIII plus XhoI and the 3.2 kbp DNA fragment bearing the *PRB1* gene was gel-purified and made blunt-ended by treatment with T4 DNA polymerase. The plasmid pUC18 was digested with BamHI, gel-purified and made blunt-ended by treatment with T4 DNA polymerase. The resulting vector fragment was ligated with the above *PRB1* gene fragment to yield the plasmid pUC18-PRB1. Plasmid YEp6, which contains the *HIS3* gene, was digested with BamHI. The resulting 1.7 kbp BamHI fragment bearing the functional HIS3 gene was gel-purified and then made blunt-ended by treatment with T4 DNA polymerase. Plasmid pUC18-PRB1 was digested with EcoRV plus Ncol which cut within the *PRB1* coding sequence and removes the protease B active site and flanking sequence. The 5.7 kbp EcoRV-NcoI fragment bearing the residual 5' and 3'-portions of the *PRB1* coding sequence in pUC18 was gel-purified. made blunt-ended by treatment with T4 DNA polymeraae, dephosphorylated with calf intestine alkaline phosphatase, and ligated with the blunt-ended *HIS3* fragment described above. The resulting plasmid (designated pUC18-prb 1::HIS3, stock #1245) contains the functional *HIS3* gene in place of the portion of the *PRB1* gene which had been deleted above.

### EXAMPLE 12

### Construction of a U9-related Yeast Strain containing disruptions of both the MNN9 and PRB1 Genes

The U9-related strain 1372 which contains a *MNN9* gene disruption was described in Example 9. Clonal isolates of strain 1372 were passaged on FOA plates (as described in Example 7) to select *ura3* mutants. A number of *ura3* isolates of strain 1372 were obtained and one particular isolate (strain 12930-190-S1-1) was selected for subsequent disruption of the *HIS3* gene. The pUC18-his3::URA3 gene disruption vector (p1505) was digested with XbaI plus EcoRI to generate a linear *his3::URA3* disruption cassette and used for transformation of strain 12930-190-S1-1 by the lithium acetate method [Methods in Enzymology, 194:290 (1991)]. Ura⁺ transformants were selected on synthetic agar medium lacking uracil, restreaked for clonal isolates on the same medium, and then replica-plated onto medium lacking either uracil or histidine to screen for those isolates that were both Ura⁺ and His-. One isolate (strain 12930-230-1) was selected for subsequent disruption of the *PRB1* gene. The *PRB1* gene disruption vector (pUC18-prb1::HIS3, stock #1245) was digested with Sad plus Xbal to generate a linear *prb1::HIS3* disruption cassette and used for transformation of strain 12930-230-1 by the lithium acetate method. His⁺ transformants were selected on agar medium lacking histidine and restreaked on the same medium for clonal isolates. Genomic DNA was prepared from a number of the resulting His⁺ isolates, digested with EcoRI, and then electrophoresed on 0.8% agarose gels. Southern blot analyses were then performed using a radiolabeled 617 bp probe for the *PRB1* gene which had been prepared by PCR using the following oligodeoxynucleotide primers:

5' TGG TCA TCC CAA ATC TTG AAA 3'
5' CAC CGT AGT GTT TGG AAG CGA 3'

Eleven isolates were obtained which showed the expected hybridization of the probe with a 2.44 kbp *prb1::HIS3* DNA fragment. This was in contrast to hybridization of the probe with the 1.59 kbp fragment for the wild-type *PRB1* gene. One of these isolates containing the desired *prb1::HIS3* disruption was selected for further use and was designated strain #1558.

### EXAMPLE 13

### Expression of HPV18 L1 and L2 in Yeast

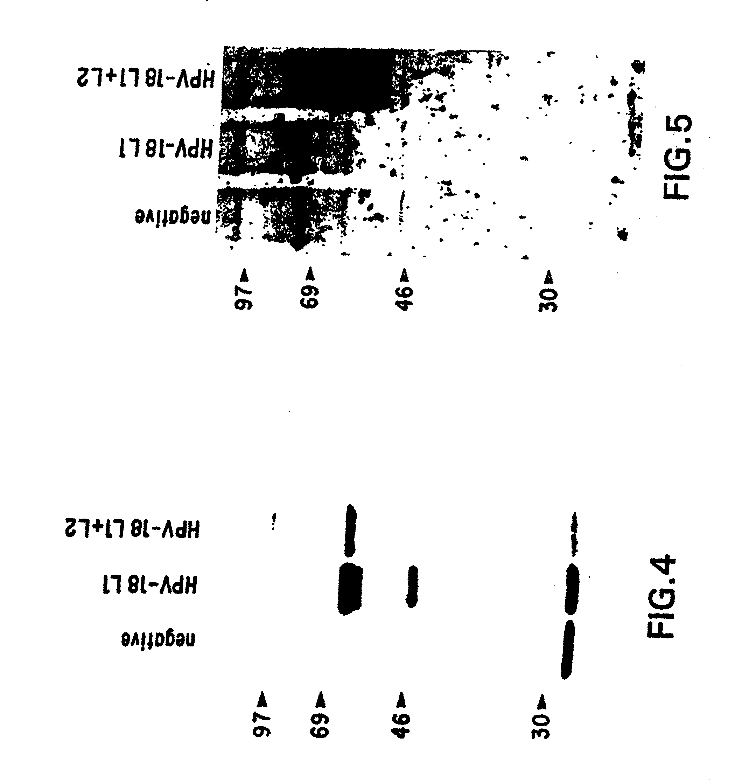

Plasmids p191-6 (PGAL1-10 + HPV18 L1) and p195-11 (pGAL1-10 + HPV18 L1 + L2) were used to transform *S. cerevisiae* strain #1558 *(MATa, leu2-04, prb1::HIS3, mnn9::URA3, adel, cir*^{*0*}*).* Clonal isolates were grown at 30°C in YEHD medium containing 2% galactose for 88 hours. After harvesting the cells, the cell pellets were broken with glass beads and cell lysates analyzed for the expression of HPV18 L1 and/or HPV18 L2 protein by immunoblot analysis. Samples containing 25 ug of total cellular protein were electrophoresed through 10% Tris-Glycine gels (Novex, Inc.) under denaturing conditions and electroblotted onto nitrocellulose filters. L1 protein was immunodetected using rabbit antiserum raised against a *trpE-*HPV11 L1 fusion protein as primary antibody (Brown *et al.,* 1994, *Virology* 201:46=54) and horseradish peroxidase (HRP)-linked donkey anti-rabbit IgG (Amersham, Inc.) as secondary antibody. The fibers were processed using the chemiluminescent ECL™ Detection Kit (Amersham, Inc.). A 50-55 KDa L 1 protein band was detected in both the L 1 and L1 + L2 coexpressor yeast clones (strains 1725 and 1727, respectively) and not in the negative control (pGAL1-10 without L1 or L2 genes) (Figure 4).

The HPV 18 L2 protein was detected by Western analysis using goat polyclonal antiserum raised against a trpE-HPV18 L2 fusion protein as primary antibody followed by HRP-conjugated, rabbit anti-goat IgG (Kirkegaard and Perry Laboratories. Gaithersburg, MD). The filters were processed as described above. The L2 protein was detected as a 75 kDa protein band in the L1 + L2 coexpressor yeast clone (strain 1727) but not in either the negative control or the L1 expressor clone (Figure 5).

### EXAMPLE 14

### Fermentation of HPV 18 L1 (strain 1725) and 18 L1 +ΔL2 (strain 1727).

Surface growth of a plate culture of strains 1725 and 1727 was aseptically transferred to a leucine-free liquid medium containing (per L): 8.5 g Difco yeast nitrogen base without amino acids and ammonium sulfate; 0.2 g adenine; 0.2 g uracil; 10 g succinic acid; 5 g ammonium sulfate; 40 g glucose; 0.25 g L-tyrosine; 0.1 g L-arginine; 0.3 g L-isoleucine; 0.05 g L-methionine; 0.2 g L-tryptophan; 0.05 g L-histidine; 0.2 g L-lysine; 0.3 g L-phenylalanine; this medium was adjusted to pH 5.0-5.3 with NaOH prior to sterilization. After growth at 28°C, 250 rpm on a rotary shaker, frozen culture vials were prepared by adding sterile glycerol to a final concentration of 17% (w/v) prior to storage at - 70°C (1 mL per cryovial). Inocula were developed in the same medium (500 mL per 2-L flask) and were started by transferring the thawed contents of a frozen culture vial and incubating at 28°C. 250 rpm on a rotary shaker for 29 hr. Fermentations of each strain used a New Brunswick SF-1 16 fermentor with a working volume of 10 L after inoculation. The production medium contained (per L): 20 g Difco yeast extract; 10 g Sheffield HySoy peptone; 20 g glucose; 20 g galactose: 0.3 mL Union Carbide UCON LB-625 antifoam; the medium was adjusted to pH 5.3 prior to sterilization. The entire contents (500 mL) of the 2-L inoculum flask was transferred to the fermentor which was incubated at 28°C. 5 L air per min, 400 rpm, 3.5 psi pressure. Agitation was increased as needed to maintain dissolved oxygen levels of greater than 40% of saturation. Progress of the fermentation was monitored by off-line glucose measurements (Beckman Glucose 2 Analyzer) and on-line mass spectrometry (Perkin-Elmer 1200). After incubation for 66 hr, cell densities of 9.5 to 9.7 g dry cell weight per L were reached. The cultures were concentrated by hollow fiber filtration (Amicon H5MP01-43 cartridge in an Amicon DC-10 filtration system) to ca. 2 L. diafiltered with 2 L phosphate-buffered saline, and concentrated further (to ca. 1L) before dispensing into 500-mL centrifuge bottles. Cell pellets were collected by centrifugation at 8,000 rpm (Sorval GS-3 rotor) for 20 min at 4°C. After decanting the supernatant, the pellets (total 191 to 208 g wet cells) were stored at - 70°C until use.

### EXAMPLE 15

### Purification of Recombinant HPV Type 18 L1 Capsid Proteins

All steps performed at 4°C unless noted.

Cells were stored frozen at -70°C. Frozen cells (wet weight = 126 g) were thawed at 20-23°C and resuspended in 70 mL "Breaking Buffer" (20 mM sodium phosphate, pH 7.2, 100 mM NaCl). The protease inhibitors PMSF and pepstatin A were added to final concentrations of 2 mM and 1.7 µM, respectively. The cell slurry was broken at a pressure of approximately 16,000 psi by 4 passes in a M110-Y Microfluidizer (Microfluidics Corp., Newton, MA). The broken cell slurry was centrifuged at 12,000 x g for 40 min to remove cellular debris. The supernatant liquid containing L 1 antigen was recovered.

The supernatant liquid was diluted 1:5 by addition of Buffer A (20 mM MOPS, pH 7.0) and applied to an anion exchange capture column (9.0 cm ID x 3.9 cm) of Fractogel® EMD TMAE-650 (M) resin (EM Separations, Gibbstown, NJ) equilibrated in Buffer A. Following a wash with Buffer A, the antigen was eluted with a gradient of 0-1.0 M NaCl in Buffer A. Column fractions were assayed for total protein by the Bradford method. Fractions were then analyzed at equal total protein loadings by Western blotting and SDS-PAGE with silver stain detection.

TMAE fractions showing comparable purity and enrichment of L1 protein were pooled. The antigen was concentrated by ammonium sulfate fractionation. The solution was adjusted to 35% saturated ammonium sulfate by adding solid reagent while gently stirring over 10 min. The sample was placed on ice and precipitation allowed to proceed for 4 hours. The sample was centrifuged at 16.000 x g for 45 min. The pellet was resuspended in 20.0 mL PBS (6.25 mM Na phosphate. pH 7.2. 150 mM NaCl).

The resuspended pellet was chromatographed on a size exclusion column (2.6 cm ID x 89 cm) of Sephacryl 500 HR resin (Pharmacia, Piscataway, NJ). Running buffer was PBS. Fractions were analyzed by western blotting and SDS-PAGE with silver stain detection. The purest fractions were pooled. The resulting pool was concentrated in a 50 mL stirred cell using 43 mm YM-100 flat-sheet membranes (Amicon, Beverly, MA) at a N₂ pressure of 4-6 psi.

Final product was analyzed by western blotting and SDS-PAGE with colloidal Coomassie detection. The L 1 protein was estimated to be 50-60% homogeneous. The identity of L1 protein was confirmed by western blotting. The final product was aliquoted and stored at -70°C. This process resulted in a total of 12.5 mg protein.

### Bradford Assay for Total Protein

Total protein was assayed using a commercially available Coomassie Plus® kit (Pierce, Rockford, IL). Samples were diluted to appropriate levels in Milli-Q-H₂O. Volumes required were 0.1 mL and 1.0 mL for the standard and microassay protocols, respectively. For both protocols, BSA (Pierce, Rockford, IL) was used to generate the standard curve. Assay was performed according to manufacturer's recommendations. Standard curves were plotted using CricketGraph ® software on a Macintosh IIci computer.

### SDS-PAGE and Western Blot Assays

All gels, buffers, and electrophoretic apparatus were obtained from Novex (San Diego, CA) and were run according to manufacturer's recommendations. Briefly, samples were diluted to equal protein concentrations in Milli-Q-H₂O and mixed 1:1 with sample incubation buffer containing 200 mM DTT. Samples were incubated 15 min at 100°C and loaded onto pre-cast 12% Tris-glycine gels. The samples were electrophoresed at 125V for I hr45 min. Gels were developed using either silver staining by a variation of the method of Heukeshoven and Demick | *Electrophoresis. 6 (1985) 103-112*| or colloidal Coomassie staining using a commercially obtained kit (Integrated Separation Systems, Natick, MA).

For western blots, proteins were transferred to PVDF membranes at 25V for 40 min. Membranes were washed with Milli-Q-H2O and air-dried. Primary antibody was polyclonal rabbit antiserum raised against a TrpE-HPV 11L1 fusion protein (gift of Dr. D. Brown). Previous experiments had shown this antiserum to cross react with HPV type 18 L1 on western blots. The antibody solution was prepared by dilution of antiserum in blotting buffer (5% non-fat milk in 6.25 mM Na phosphate, pH 7.2, 150 mM NaCl, 0.02% NaN₃). Incubation was for at least 1 hour at 20-23°C. The blot was washed for 1 min each in three changes of PBS (6.25 mM Na phosphate, pH 7.2, 150 mM NaCl). Secondary antibody solution was prepared by diluting goat anti-rabbit IgG alkaline phosphatase-linked conjugate antiserum (Pierce, Rockford, IL) in blotting buffer. Incubation proceeded under the same conditions for at least 1 hour. Blots were washed as before and detected using a 1 step NBT/BCIP substrate (Pierce, Rockford; IL).

### EXAMPLE 16

### Electron Microscopic Studies

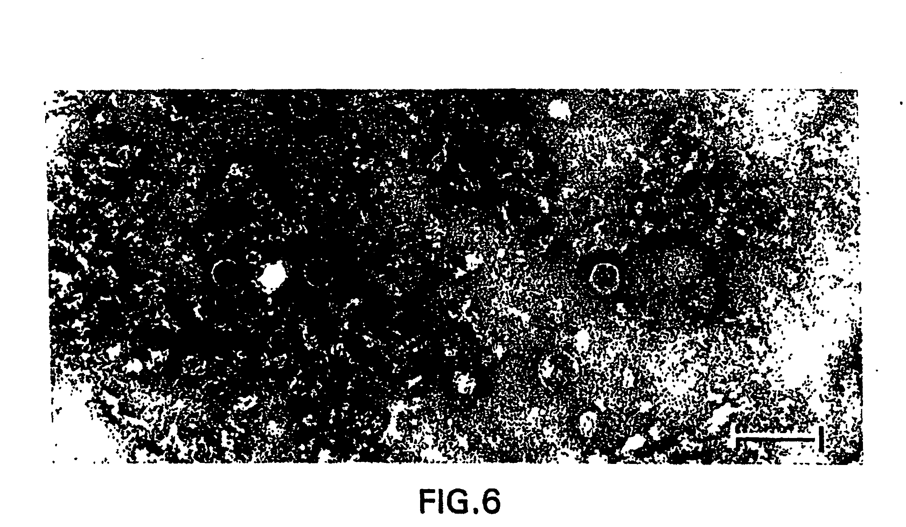

For EM analysis (Structure Probe, West Chester, PA), an aliquot of each sample was placed on 200-mesh carbon-coated copper grids. A drop of 2% phosphotungstic acid (PTA), pH 7.0 was placed on the grid for 20 seconds. The grids were allowed to air dry prior to transmission EM examination. All microscopy was done using a JEOL 100CX transmission electron microscope (JEOL USA, Inc.) at an accelerating voltage of 100 kV. The micrographs generated have a final magnification of 100.000x. Virus-like particles were observed in the 50-55 nm diameter size range in the yeast sample harboring the HPV18L1 expression plasmid (Figure 6). No VLPs were observed in the yeast control samples.

### EXAMPLE 17

### Sub-cloning of the HPV18 cDNA into expression vectors

The cDNA encoding HPV18 is sub-cloned into several vectors for expression of the HPV 18 protein in transfected host cells and for *in vitro* transcription/translation. These vectors include pBluescript II SK+ (where expression is driven by T7 or T3 promoters) pcDNA I/Amp (where expression is driven by the cytomegalovirus (CMV) promoter), pSZ9016-1 (where expression is driven by the HIV long terminal repeat (LTR) promoter) and the baculovirus transfer vector pAcUW51 (PharMingen, Inc.) (where expression is driven by the polyhedrin (PH) promoter) for producing recombinant baculovirus containing the HPV 18 encoding DNA sequence.
a) pBluescript II SK+:HPV18. The full length HPV18 cDNA clone is retrieved from lambda bacteriophage by limited *Eco* RI digestion and ligated into *Eco* RI-cut, CIP-treated pBluescript II SK+. Separate subclones are recovered in which the sense orientation of HPV 18 followed either the T7 or T3 promoters.
b) pcDNA I/Am:HPV18. To facilitate directional cloning, HPV 18 is excised from a purified plasmid preparation of pBluescript II SK+:HPV18 in which the HPV18 DNA sequence is downstream of the T7 promoter using *Eco* RV and *Xba* I. The resulting *Eco* RV, *Xhu* I HPV18 fragment is purified and ligated into *Eco* RV-cut. *Xba* I-cut, CIP-treated pcDNA I/Amp such that the HPV18 encoding DNA is downstream of the CMV promoter.
c) pSZ9016-1:HPV18. HPV18 is excised from pBluescript II SK+:HPV18 by limited *Eco* RI digestion and subsequent purification of the 1.3 Kb fragment from agarose gels. The resulting *Eco* RI HPV18 fragment is ligated into *Eco* RI-cut, CIP-treated pSZ9016-1. Subclones are selected in which the sense orientation of HPV 18 is downstream of the HIV LTR promoter.
d) pAcUW51:HPV18 L1, The full-length HPV18 L1 ORF was amplified by PCR from clone #187-1 using oligonucleotide primers providing flanking BgIII sites. The L1 gene was inserted into the BamHI site of the baculovirus transfer vector. pAcUW51 (PharMingen. Inc.), under control of the polyhedrin promoter. Recombinant baculoviruses were generated containing the HPV18 L1 expression cassette according to the procedures described in the Pharmingen Manual. Recombinant clones were purified by limiting dilution and dot blot hybridization.

### EXAMPLE 18

### Expression Of The HPV 18 Polypeptide By In Vitro Transcription/Translation And By Transfection Into Host Cells

Vectors containing HPV DNA sequences are used to drive the translation of the HPV 18 polypeptide in rabbit reticulocyte lysates, mammalian host cells, and in baculovirus infected insect cells. The experimental procedures are essentially those outlined in the manufacturers' instructions.
a) *In vitro* Transcription/Translation, pBluescript III SK+:HPV 18 plasmid DNA (with HPV 18 in the T7 orientation) is linearized by *Bam* HI digestion downstream of the HPV18 insert. The linearized plasmid is purified and used as a template for run-off transcription using T7 RNA polymerase in the presence of m7G(5')ppp(5')G. The resulting capped HPV18 transcripts are purified by LiCl precipitation and used to drive the translation of HPV 18 in nuclease-pretreated rabbit reticulocyte lysate in the presence of L-[³⁵S]methionine.
b) Expression in Mammalian Cells. The HPV18 protein is expressed in mammalian host cells following transfection with either pcDNA I/Amp:HPV18 (under control of the CMV promoter) or pSZ9016-1:HPV18 (under control of the HIV LTR promoter). In the latter case (pSZ9016-1:HPV18), cells are co-transfected with the TAT expressing plasmid pSZ90161:TAT. For both HPV18 expression plasmids. COS-7 cells are transfected using either DEAE-dextran or lipofection with Lipofectamine (BRL).
c) Expression in Insect Cells. The HPV18 L1-containing baculovirus transfer vector pAcUW51:HPV18 L1 is used to produce recombinant baculovirus *(Autographa californica)* by *in vivo* homologous recombination. Epitope tagged HPV 18 L1 is then expressed in Sf9 *(Spodoptera frugiperda)* insect cells grown in suspension culture following infection with the HPV 18 - containing recombinant baculovirus.

### EXAMPLE 19

Compounds that affect HPV 18 activity may be detected by a variety of methods. A method of identifying compounds that affect HPV18 comprises:
(a) mixing a test compound with a solution containing HPV 18 to form a mixture;
(b) measuring HPV 18 activity in the mixture; and
(c) comparing the HPV 18 in the mixture to a standard.

Compounds that affect HPV 18 activity may be formulated into pharmaceutical compositions. Such pharmaceutical compositions may be useful for treating diseases or conditions that are characterized by HPV 18 infection.

### EXAMPLE 20

DNA which is structurally related to DNA encoding HPV 18 is detected with a probe. A suitable probe may be derived from DNA having all or a portion of the nucleotide sequence of Figure 1 or Figure 3, RNA encoded by DNA having all or a portion of the nucleotide sequence of Figure 1 or Figure 3 or degenerate oligonucleotides derived from a portion of the sequence of Figure 1 or Figure 3.

## Claims

1. Virus-like particles comprised of recombinant L1 protein or recombinant L1 + L2 proteins of human papillomavirus 18, the virus-like particles being at least 60% pure, wherein the L1 protein is shown in Figure 1 and the L2 protein is shown in Figure 3.

2. The virus-like particles of Claim 1 wherein the recombinant L1 protein or the recombinant L1 + L2 proteins is produced in yeast.

3. A method of producing the virus-like particles of Claim 1 comprising:
(a) transforming yeast with a recombinant DNA molecule encoding papillomavirus L1 protein or papillomavirus L1 + L2 proteins;
(b) cultivating the transformed yeast under conditions that permit expression of the recombinant DNA molecule to produce the recombinant papillomavirus protein; and
(c) isolating the recombinant papillomavirus protein to produce virus-like particles of Claim 1.

4. Recombinant papillomavirus protein produced by the method of Claim 3.

5. A vaccine comprising the virus-like particles of Claim 1.

6. Pharmaceutical compositions comprising the virus-like particles of Claim 1 and a pharmaceutically acceptable excipient.

7. An isolated and purified HPV18 L1 DNA molecule shown in Figure 1, or an HPV18 L2 DNA molecule shown in Figure 3.

8. An expression vector for expression of the DNA molecule of Claim 7 in a host.

9. An essentially purified protein encoded by the DNA molecule of Claim 7.

10. A process for expression of a human papillomavirus type 18 protein in a host, comprising:
(a) transferring the expression vector of Claim 9 into a suitable host; and
(b) culturing the host of step (a) under conditions which allow expression of the human papillomavirus type 18 protein from the expression vector.

11. A pharmaceutical composition comprising a compound selected from the DNA molecule of Claim 7, peptides encoded by the DNA molecule of Claim 7, RNA complementary to the DNA molecule of Claim 7, or combinations thereof and a pharmaceutically acceptable carrier.

## Patentansprüche

1. Virusähnliche Partikel, umfassend rekombinantes L1-Protein oder rekombinante L1 - + L2-Proteine von Human-Papillomavirus 18, wobei die virusähnlichen Partikel zu mindestens 60 % rein sind, das L1-Protein in Figur 1 gezeigt ist und das L2-Protein in Fig. 3 gezeigt ist.

2. Virusähnliche Partikel nach Anspruch 1, wobei das rekombinante L1-Protein oder die rekombinanten L1- + L2-Proteine in Hefe produziert wird/werden.

3. Verfahren zur Herstellung der virusähnlichen Partikel nach Anspruch 1, umfassend:
(a) Transformation von Hefe mit einem rekombinanten DNA-Molekül, das für Papillomavirus-L1-Protein oder Papillomavirus-L1- + L2-Proteine kodiert;
(b) Kultivierung der transformierten Hefe unter Bedingungen, welche die Expression des rekombinanten DNA-Moleküls erlauben, um das rekombinante Papillomavirus-Protein herzustellen; und
(c) Isolierung des rekombinanten Papillomavirus-Proteins, um virusähnliche Partikel nach Anspruch 1 herzustellen.

4. Rekombinantes Papillomavirus-Protein, hergestellt nach dem Verfahren nach Anspruch 3.

5. Vakzin, umfassend die virusähnlichen Partikel nach Anspruch 1.

6. Pharmazeutische Zusammensetzungen, umfassend die virusähnlichen Partikel nach Anspruch 1 und einen pharmazeutisch annehmbaren Exzipienten.

7. Isoliertes und gereinigtes HPV18-L1-DNA-Molekül, gezeigt in Fig. 1, oder HPV18-L2-DNA-Molekül, gezeigt in Fig. 3.

8. Expressionsvektor zur Expression des DNA-Moleküls nach Anspruch 7 in einem Wirt.

9. Im wesentlichen gereinigtes Protein, kodiert von dem DNA-Molekül nach Anspruch 7.

10. Verfahren zur Expression eines Human-Papillomavirus-Typ 18-Proteins in einem Wirt, umfassend:
(a) Überführung des Expressionsvektors nach Anspruch 9 in einen geeigneten Wirt; und
(b) Kultivierung des Wirts von Schritt (a) unter Bedingungen, welche die Expression des Human-Papillomavirus-Typ 18-Proteins von dem Expressionsvektor erlauben.

11. Pharmazeutische Zusammensetzung, umfassend eine Verbindung, die aus dem DNA-Molekül nach Anspruch 7, Peptiden, die von dem DNA-Molekül nach Anspruch 7 kodiert werden, RNA, die zu dem DNA-Molekül nach Anspruch 7 komplementär ist, oder Kombinationen davon ausgewählt ist, und einen pharmazeutisch annehmbaren Träger.

## Revendications

1. Particules de nature virale comprenant une protéine L1 recombinante ou des protéines L1 + L2 recombinantes du virus du papillome de type 18 humain, les particules de nature virale étant pures à au moins 60%, dans lesquelles la protéine L1 est représentée sur la Figure 1 et la protéine L2 est représentée sur la Figure 3.

2. Particules de nature virale selon la revendication 1, dans lesquelles la protéine L1 recombinante ou les protéines L1 + L2 recombinantes sont produites dans une levure.

3. Procédé de production des particules de nature virale selon la revendication 1, comprenant :
(a) la transformation d'une levure avec une molécule d'ADN recombinant codant pour la protéine L1 du virus du papillome ou pour les protéines L1 + L2 du virus du papillome ;
(b) la culture de la levure transformée dans des conditions qui permettent l'expression de la molécule d'ADN recombinant pour produire la protéine recombinante du virus du papillome ; et
(c) l'isolement de la protéine recombinante du virus du papillome pour produire des particules de nature virale selon la revendication 1.

4. Protéine recombinante du virus du papillome produite par le procédé selon la revendication 3.

5. Vaccin comprenant les particules de nature virale selon la revendication 1.

6. Compositions pharmaceutiques comprenant les particules de nature virale selon la revendication 1 et un excipient pharmaceutiquement acceptable.

7. Molécule d'ADN de L1 du HPV18 isolée et purifiée, représentée sur la Figure 1, ou molécule d'ADN de L2 du HPV18, représentée sur la Figure 3.

8. Vecteur d'expression pour l'expression de la molécule d'ADN selon la revendication 7 dans un hôte.

9. Protéine essentiellement purifiée codée par la molécule d'ADN selon la revendication 7.

10. Procédé pour l'expression d'une protéine du virus du papillome de type 18 humain dans un hôte, comprenant :
(a) le transfert du vecteur d'expression selon la revendication 9 dans un hôte approprié ; et
(b) la culture de l'hôte de l'étape (a) dans des conditions qui permettent l'expression de la protéine du virus du papillome de type 18 humain à partir du vecteur d'expression.

11. Composition pharmaceutique comprenant un composé choisi parmi la molécule d'ADN selon la revendication 7, des peptides codés par la molécule d'ADN selon la revendication 7, l'ARN complémentaire de la molécule d'ADN selon la revendication 7, ou des associations de ceux-ci et un véhicule pharmaceutiquement acceptable.
